# EUROPÄISCHE PATENTSCHRIFT

(11) **EP 1 651 244 B1**
(45) Veröffentlichungstag und Bekanntmachung des Hinweises auf die Patenterteilung: **29.08.2007**
(21) Anmeldenummer: 05757932.8
(22) Anmeldetag: 24.06.2005
(51) Int. Cl.: A61K 36/00, A61P 31/18

(54) **Verwendung von Extrakten aus Wurzeln von Pelargonium sidoides und/oder Pelargonium Reniforme**
Use of extracts from roots of Pelargonium sidoides and/or Pelargonium Reniforme
Utilisation d'extraits de racines de Pélargonium sidoides et/ou Pélargonium réniforme

(30) Priorität: 05.07.2004 DE 102004032439
(43) Veröffentlichungstag der Anmeldung: 03.05.2006
(73) Patentinhaber: Dr. Willmar Schwabe GmbH & Co. KG, 76227 Karlsruhe (DE)
(72) Erfinder: KOCH, Egon, 76229 Karlsruhe (DE); HAUER, Hermann, 76228 Karlsruhe (DE); STUMPF, Karl-Heinz, 76228 Karlsruhe (DE)
(74) Vertreter: Adam, Holger
(86) Internationale Anmeldenummer: PCT/EP2005/006853
(87) Internationale Veröffentlichungsnummer: WO 2006/002837

(56) Entgegenhaltungen:
- WO-A-96/05849
- WO-A-03/028746
- DE-B3- 10 350 338
- KOLODZIEJ H ET AL: "PELARGONIUM SIDOIDES DC. NEUESTE ERKENNTNISSE ZUM VERSTAENDNIS DES PHYTOTHERAPEUTIKUMS UMCKALOABO" ZEITSCHRIFT FUER PHYTOTHERAPIE, STUTTGART, DE, Bd. 19, Nr. 3, 1998, Seiten 141-151, XP008011448
- HAIDVOGL M ET AL: "AKUTE BRONCHITIS IM KINDESALTER" ZEITSCHRIFT FUER PHYTOTHERAPIE, STUTTGART, DE, Bd. 17, Nr. 5, 1996, Seiten 300,303-304,307, XP008011447

## Beschreibung

Die Erfindung betrifft die Verwendung von Extrakten aus Wurzeln von Pelargonium sidoides und/oder Pelargonium reniforme zur Herstellung eines Arzneimittels zur Behandlung von AIDS und AIDS-assoziierten Infektionen verursacht durch andere Viren, Bakterien, insbesondere Mykobakterien, Pilze und Parasiten.

Etwa ein Drittel der Weltbevölkerung leidet an Tuberkulose und diese Erkrankung, die durch eine Infektion mit Mycobakterium tuberculosis ausgelöst wird, zählt weltweit zu den zehn wichtigsten Todesursachen. Die Häufigkeit von Infektionen mit M. tuberculosis hat in den vergangen drei Jahrzehnten durch die Ausbreitung der HIV/AIDS-Epidemie und die Entwicklung von Resistenzen gegenüber Chemotherapeutika insbesondere in Afrika und Südostasien dramatisch zugenommen. Die Schwächung der Immunabwehr durch die Infektion mit HIV-Viren schafft in den Patienten ideale Bedingungen für die Entstehung von Koinfektionen, wie z. B. mit Bakterien (z. B. M. tuberculosis), Viren (z. B. Herpesviren), Parasiten und Pilze. Schätzungen gehen davon aus, dass die gleichzeitige Infektion mit Tuberkulose für etwa ein Drittel aller Todesfälle bei AIDS-Patienten verantwortlich ist. Während in den industrialisierten Ländern durch die bessere hygenische und medizinische Versorgung sowie den Einsatz von antiretroviralen Medikamenten die Lebenserwartung von AIDS-Patienten wesentlich verbessert werden konnte, stehen diese Arzneimittel Infizierten in Entwicklungsländern häufig, nicht zuletzt aufgrund der hohen Kosten für diese Medikamente, nicht zur Verfügung. Es besteht deshalb ein dringender Bedarf, kostengünstige Alternativen für Patienten in diesen Regionen zu entwickeln. Die Aufgabe der vorliegenden Erfindung liegt daher in der Bereitstellung von Verbindungen und Arzneimitteln zur Behandlung von AIDS.

Diese Aufgabe wurde erfindungsgemäß durch die Verwendung von Extrakten aus Pelargonium sidoides und/oder Pelargonium reniforme zur Herstellung eines Arzneimittels zur Behandlung von AIDS gelöst.

Pelargonium sidoides und Pelargonium reniforme sind Geraniaceen, die im südlichen Afrika traditionell als Arzneimittel verwendet wurden. Die volksmedizinische Verwendung umfasst die Behandlung von Diarrhöe, gastrointestinalen Beschwerden, Dysmenorrhoe und Lebererkrankungen. Üblich ist aber auch die Anwendung bei Atemwegserkrankungen und insbesondere bei Lungentuberkulose. Für Zubereitungen aus Pelargonium sidoides/reniforme ist die Bezeichnung "Umckaloabo" geläufig, die sich aus Begriffen der Zulu-Sprache für charakteristische Tuberkulose-Symptome ableitet, wie z. B. Husten, Auswurf, Fieber und körperliche Schwäche bzw. schneidende Schmerzen in der Brustgegend. Auch in Europa war die Verwendung der Umckadroge zur adjuvanten Behandlung der Tuberkulose unter der Bezeichnung Stevenskur zu Beginn dieses Jahrhunderts verbreitet. Entsprechend der traditionellen Verwendung ist **heute ein kommerzieller Extrakt aus Pelargonium sidoides/reniforme auf dem Markt, der** vor allem zur Behandlung verschiedener akuter und chronischer Erkrankungen der Atemwege und des Hals-Nasen-Ohrenbereichs wie Rhinopharyngitis, Tonsillitis, Sinusitis und Bronchitis eingesetzt wird.

Es wurde jetzt überraschend beobachtet, dass Extrakte aus den Wurzeln von Pelargonium sidoides/reniforme nicht nur über potente antimykobakterielle und lymphoproliferative Eigenschaften verfügen, sondern zusätzlich effektiv die Infektion von humanen Lymphozyten mit dem Human Immundefizienz Virus Typ 1 (HIV-1) hemmen. Zubereitungen aus Pelargonium sidoides/reniforme stellen deshalb interessante Alternativen zur Behandlung von AIDS-Infektionen und AIDS-assozierten Infektionen dar, insbesondere von bakteriellen Infektionen wie z. B. durch M. tuberculosis, atypische Mykobakterien (z. B. M. avium intraclulare), Enteritis-Salmonellen oder fakultative pathogene Bakterien (z. B. Streptokokken, Pneumokokken, Staphylokokken oder Hämophilus), von viralen Infektionen wie z. B. durch Herpesviren (z. B. Varicella-Zoster, Herpes simplex), Cytomegalieviren oder Hepatitisviren, von Infektionen durch Pilze wie z. B. Candida, Cryptococcus oder Aspergillus, sowie von parasitären Infektionen z. B. durch Pneumocystis carinii oder Toxoplasmen.

Extrakte aus Pelargonien oder deren Pflanzenteilen können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemp. bis 60 °C unter gelinder bis heftiger Durchmischung oder durch Perkolation innerhalb von 10 Min. bis 24 Std. erhalten werden. Bevorzugte Extraktionslösungsmittel sind dabei Gemische von Ethanol und Wasser, besonders bevorzugt im Verhältnis **Ethanol / Wasser = 10 / 90 bis 15 / 85 (w/w). Zur Anreicherung** wirksamkeitsbestimmender Komponenten können weitere Konzentrierungsschritte durchgeführt werden wie z. B. flüssig-flüssig-Verteilung mit z. B. 1-Butanot/Wasser oder Ethylacetat/Wasser, Adsorption-Desorption an lonenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und /oder reduziertem Druck oder durch Gefriertrocknung.

Die erfindungsgemäß verwendeten Extrakte können in Form von Pulvern, Granulaten, Tabletten, Dragees oder Kapseln oder auch als Lösung, wie sie z. B. bei der Extraktion direkt anfällt, vorzugsweise oral verabreicht werden.

Zur Herstellung von Tabletten wird der Extrakt mit geeigneten pharmazeutisch verträglichen Hilfstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxymethylpropylcellulose, Polyethylenglykol, Farbstoffen (z. B. Titandioxid, Eisenoxid) und Talkum versehen werden.

Die erfindungsgemäß verwendeten Extrakte können auch, ggf. unter Zusatz von Hilfsstoffen wie z. B. Stabilisatoren, Füllmittel etc., in Kapseln abgefüllt werden. Die Dosierung erfolgt dabei so, dass pro Tag 2 bis 1000 mg, bevorzugt 10 bis 200 mg Extrakt zugeführt werden.

Die Wirksamkeit von ethanolisch-wäßrigen Extrakten aus Pelargonium sidoides bei AIDS-Infektionen und bei AIDS-assozierten Infektionen wie z. B. Tuberkulose wird durch die nachstehenden pharmakologischen Experimente belegt.

### Prüfung auf Anti-HIV-Aktivität

Für die Untersuchungen wurde der HIV-1-Stamm HTLV-III_{RF} (National Institute for Biological Standards and Control, Hertfordshire, UK) verwendet. Es handelt sich dabei um einen synzytieninduzierenden Virusstamm (SI-Variante). Die in der Ausgangssuspension vorhandene Menge infektiöser HIV Partikel wurde im klassischen Titrationsverfahren gemessen. Dabei wird die höchste Verdünnung der Virussuspension ermittelt, die bei 50 % der Indikatorzellen einen zytopathologischen Effekt (CPE) verursacht (50 % tissue culture infective doses pro ml (TCLD50 /ml)). Hierzu wurden serielle Verdünnungen der Stammsuspension erstellt (1:10, 1:100, 1:1.000 usw.) und je 50 µl auf Indikatorzellen (s. u.) in 96-Loch-Platten (im achtfach Parallelansatz) gegeben. Die Ablesung erfolgte mikroskopisch nach 5 Tagen durch zwei unabhängige Untersucher. Als Indikatorzellen wurden C8166-Zellen (humane lymphoide Zellen; National Institute for Biological Standards and Control, Hertfordshire, UK) in Suspensionskultur eingesetzt.

Zum Nachweis der "antiviralen" Aktivität wurden acht verschiedene, definierte Konzentrationen (100; 50; 25; 12,5; 6; 3; 1,5; 0,75 µg/ml) von Pelargonium-Extrakt mit einer definierten Menge HIV-Virus (4.000 TCID₅₀/50 µl) und Zellen (100.000 Zellen/50 µl) gemischt. Nach 5-tägiger Inkubation im Brutschrank bei 37 °C erfolgte die Auswertung durch mikroskopische Analyse auf virusspezifische CPEs. Die Versuche wurden als Einfachansatz in jeweils Achtfachbestimmung durchgeführt.

Die Bestimmung der Zytotoxizität erfolgte mittels MTT-Test. Methodisch wurde dabei analog dem oben beschriebenen Prozedere vorgegangen. Die Auswertung der Zellen erfolgte nach 5 Tagen. Die Zytotoxizität wird angegeben als Substanzkonzentration, bei der ca. 50 % der Indikatorzellen durch toxische Einflüsse zerstört sind (TC₅₀).

Die Berechnung der virusinhibitorischen Konzentration (IC₅₀) erfolgt mittels linearer Regression unter Verwendung eines Computerprogrammes. Der IC₅₀-Wert stellt die Substanzkonzentration da, bei der eine Reduktion der Virus-Replikation um 50 % erreicht wird. Zusätzlich angegeben ist der therapeutische Index (TI), d. h. der Quotient aus TC₅₀ und IC₅₀.

Der Pelargonium sidoides-Extrakt nach Beispiel 1 hemmt die Virus-Replikation halbmaximal bei einer Konzentration von 28 µg/ml. Für die zytotoxischen Wirkungen (IC₅₀-Konzentration) wurde ein Wert von 66 µg/ml ermittelt, woraus sich ein therapeutischer Index von 2,4 ergibt.

### Prüfung auf antimykobakterielle Aktivität

Zur Prüfung auf antimykobakterielle Wirksamkeit wurde Mycobacterium ranae verwendet, ein nicht humanpathogenes Mykobakterium, das über eine ähnliche Empfindlicheit gegenüber Chemotherapeutika verfügt wie M. tuberculosis. Zur Bestimmung der minimalen Hemmkonzentration (MHK) wurde Suspensions-Verdünnungsmethode verwendet. Der Pelargonium sidoides-Extrakt wurde in DMSO gelöst und seriell verdünnt, um die benötigten Konzentrationen zu erhalten. In 48-Well-Mikrotiterplatten, die pro Vertiefung 990 µl einer M. ranae-Suspension (ATCC 10) in einer Konzentration von 1-5x10⁵ CFU/ml Brain-Heart Infusion Broth (Difco, USA) enthielten, wurde Pelargonium-Extrakt oder das Lösungsmittel in einem Volumen von 10 µl zugegeben. Die maximale Konzentration des Lösungsmittels (DMSO) betrug 1 %. Pelargonium-Extrakt wurde bei Konzentrationen zwischen 100 und 0.03 µg/ml getestet. Alle Testansätze wurden in Duplikaten durchgeführt. Die Platten wurden für 48 h bei 37 °C inkubiert und die Hemmung des Bakterienwachstums anschließend visuell beurteilt. Für den Pelargonium sidoides-Extrakt nach Beispiel 1 wurde eine MHK von 100 µg/ml ermittelt. Gentamicin, das als positive Referenzverbindung verwendet wurde, hatte eine MHK von 0,3 µg/ml.

### Proliferation von Mauslymphozyten

Lymphozyten spielen eine zentrale Rolle in der spezifischen Immunabwehr. HIV-1-Viren infizieren vor allem CD4-Lymphozyten und der Verlust dieser für Immunreaktionen wichtigen Lymphozytenpopulation ist von entscheidener Bedeutung für die Pathogenese von AIDS und vor allem dem Aufteten der für diese Erkrankung typischen opportunistischen Infektionen. Substanzen die der Destruktion von Lymphozyten und deren verringerter Neubildung entgegenwirken, sind deshalb eine wirksame Maßnahme, um dem Defizit der zellulären Immunabwehr entgegenzuwirken.

Für die Untersuchungen der lymphoproliferativen Wirkungen von Pelargoniumextrakt wurden Milzzellen von männlichen CBA/J-Mäusene (Janvier, Le Genest, Frankreich) verwendet. Nach der Isolierung wurden die Lymphozyten in in einer Konzentration von 2 x 10⁶ Zellen/ml in komplettem RPMI 1640 Medium (supplementiert mit 10 % hitzeinaktiviertem fötalem Kälberserum 2 mM L-glutamin, 100 U/ml Penicillin, 100 µg/ml Streptomycin und 50 µM 2-Mercaptoethanol) resuspendiert. Lymphozytes (10⁵ per Vertiefung) wurden in U-Form Mikrotiterplatten (Greiner) überführt und in einem Volumen von insgesamt 200 µl kompletten RPMI 1640 Medium in Anwesenheit von verschiedenen Konzentationen von Pelargoniumextrakt oder Lösungsmittel für 72 h im Brutschrank in einer Atmosphäre von 5 % CO₂ in Luft inkubiert. Alle Testansätze wurden in Vierfachbestimmung durchgeführt. Am Ende der Inkubationsdauer wurden die Zellen mit 18.5 kBq (37 GBq/mmol spezifische Aktivität) von [Methyl-³H]Thymidin (Amersham, Germany) über einen Zeitraum von 6 h gepulst. Anschließend wurden die Zellen mit einem Zellemter (IH 110, Berthold, Bad Wildbad) auf Glasfiberfilter (Type G-10, Berthold) gesammelt und die Inkorporation der Base in neu synthetisierte DNA durch Bestimmung der Radioaktivität mit einem Proportional-Counter (LB 284RA, Berthold) bestimmt.

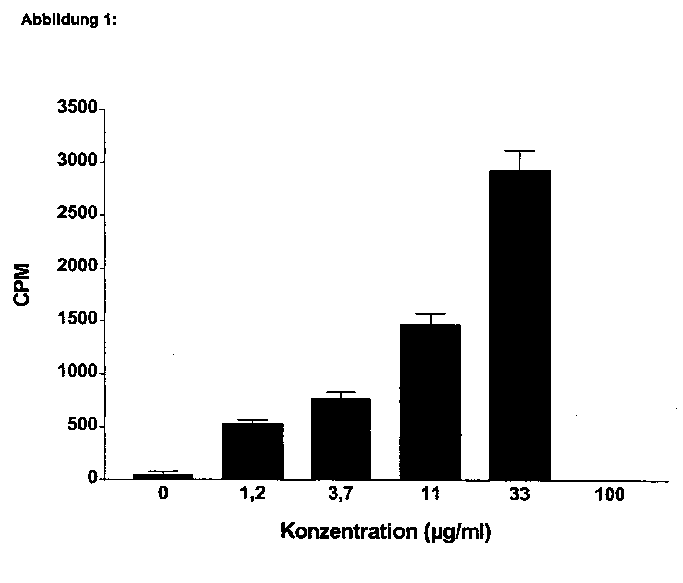

Die Ergebnisse der Untersuchungen mit dem ethanolisch-wäßrigen Pelargonium sidoides-Extrakt gemäß Beispiel 2 sind in der Abbildung 1 dargestellt. Daraus ist ersichtlich, das dieser Extrakt konzentrationsabhängig die Proliferation von Mauslymphozyten stimuliert mit einem maximalen Effekt bei 33 µg/ml.

Abbildung 1 zeigt den Effekt des ethanolisch-wäßrigen Extrakts aus Wurzeln von Pelargonium sidoides gemäß Beispiel 2 auf die spontane Proliferation von Mauslymphozyten. Die Zellproliferation wurde anhand der Inkorporation of ³H-Thymidine in neu synthetisierte DNA (counts per minute [CPM]) ermittelt.

### Beispiel 1: Extrakt aus Pelargonium sidoides

Getrocknete und gemahlene Wurzeln aus Pelargonium sidoides wurden zweimal mit der jeweils siebenfachen Gewichtsmenge Ethanol / Wasser 11 / 89 (w/w) bei 55 °C extrahiert. Nach Filtration wurde das Filtrat aufkonzentriert und bei 60 °C im Vakuum getrocknet: 106 g Trockenextrakt pro kg Pflanzenmaterial (10.6 %).

### Beispiel 2: Extrakt aus Pelargonium sidoides

Ca. 1.15 kg gemahlene Wurzeln aus Pelargonium sidoides wurden mit ca. 11.5 kg Ethanol / Wasser 11 / 89 (w/w) bei Raumtemperatur extrahiert. Nach Filtration wurde das Filtrat bei ca. 40 °C aufkonzentriert und gefriergetrocknet: 86 g (7.5 %) Trockenextrakt.

## Patentansprüche

1. Verwendung von Extrakten aus Wurzeln von Pelargonium sidoides und/oder Pelargonium reniforme zur Herstellung eines Arzneimittels zur Behandlung von AIDS.

2. Verwendung nach Anspruch 1, wobei AIDS mit Bakterien-, anderen Virus-, Pilz- und/oder Parasiteninfektionen assoziiert ist.

3. Verwendung nach Anspruch 2, wobei die bakteriellen Infektionen durch M. tuberculosis, atypische Mykobakterien umfassend M. avium intraclulare, Enteritis-Salmonellen oder fakultative pathogene Bakterien umfassend Streptokokken, Pneumokokken, Staphylokokken und Hämophilus verursacht sind.

4. Verwendung nach Anspruch 2, wobei die anderen viralen Infektionen durch Herpesviren umfassend Varicella-Zoster und Herpes simplex, Cytomegalieviren oder Hepatitisviren verursacht sind.

5. Verwendung nach Anspruch 2, wobei die Infektionen durch Pilze umfassend Candida, Cryptococcus und Aspergillus verursacht sind.

6. Verwendung nach Anspruch 2, wobei die parasitären Infektionen durch Pneumocystis carinii oder Toxoplasmen verursacht sind.

## Claims

1. Use of extracts from roots of Pelargonium sidoides and/or Pelargonium reniforme for the manufacture of a medicament for the treatment of AIDS.

2. Use according to claim 1, wherein AIDS is associated with infections by bacteria, other viruses, fungi and/or parasites.

3. Use according to claim 2, wherein the bacterial infections are caused by M. tuberculosis, atypical mycobacteria comprising M. avium intraclulare, Enteritis salmonellae or facultative pathogenic bacteria comprising streptococci, pneumococci, staphylococci and hemophilus.

4. Use according to claim 2, wherein the other viral infections are caused by herpes viruses comprising varicella-zoster and Herpes simplex, cytomegaloviruses or hepatitis viruses.

5. Use according to claim 2, wherein the infections are caused by fungi comprising candida, cryptococcus and aspergillus.

6. Use according to claim 2, wherein the parasitic infections are caused by Pneumocystis carinii or toxoplasms.

## Revendications

1. Utilisation d'extraits de racinesde Pélargonium sidoïde et/ou de Pélargonium réniforme pour la fabrication d'un médicament destiné à traiter le SIDA.

2. Utilisation selon la revendication 1, le SIDA étant associé à des infections bactériennes, à d'autres infections virales, fongiques et/ou parasitaires.

3. Utilisation selon la revendication 2, les infections bactériennes étant causées par la M. tuberculosis, par des mycobactéries atypiques comprenant la M. avium intracellulare, la Salmonella enteriditis, ou par des bactéries pathogènes facultatives comprenant des streptocoques, des pneumocoques, des staphylocoques et des Haemophilus.

4. Utilisation selon la revendication 2, les autres infections virales étant causées par des herpèsvirus comprenant le virus varicelle-zona et l'herpès simplex, des cytomégalovirus ou des virus de l'hépatite.

5. Utilisation selon la revendication 2, les infections étant causées par des champignons comprenant le Candida, le Cryptococcus et l'Aspergillus.

6. Utilisation selon la revendication 2, les infections parasitaires étant causées par le Pneumocystis carinii ou par des toxoplasmes.
